(19) Europäisches Patentamt
European Patent Office
Office européen des brevets

(11) Numéro de publication: **0 508 848 B1**

(12) **FASCICULE DE BREVET EUROPEEN**

(45) Date de publication de fascicule du brevet: **19.07.95**

(51) Int. Cl.[6]: **A61K 7/48**

(21) Numéro de dépôt: **92400651.3**

(22) Date de dépôt: **12.03.92**

(54) **Composition cosmétique pour lutter contre le vieillissement de la peau contenant en association au moins un rétinoide et au moins une dialkyl- ou trialkylxanthine.**

(30) Priorité: **20.03.91 FR 9103393**

(43) Date de publication de la demande:
**14.10.92 Bulletin 92/42**

(45) Mention de la délivrance du brevet:
**19.07.95 Bulletin 95/29**

(84) Etats contractants désignés:
**AT BE CH DE DK ES FR GB GR IT LI NL PT SE**

(56) Documents cités:
**EP-A- 0 379 367**
**WO-A-91/00726**
**DE-A- 1 810 705**
**DE-A- 1 962 999**

**WPIL / DERWENT, section Ch, semaine 8714, AN=87-097724, Derwent Publications Ltd, Londres, GB; & JP-A-62 045 527 (SHISEIDO K.K.) 27-02-1987**

(73) Titulaire: **L'OREAL**
**14, rue Royale**
**F-75008 Paris (FR)**

(72) Inventeur: **Soudant, Etienne**
**14, rue Léon Bernard**
**F-94260 Fresnes (FR)**

(74) Mandataire: **Stalla-Bourdillon, Bernard et al**
**CABINET NONY & CIE**
**29, rue Cambacérès**
**F-75008 Paris (FR)**

**EP 0 508 848 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet une composition cosmétique destinée à lutter contre le vieillissement de la peau et en vue d'en améliorer son aspect esthétique contenant en association un rétinoïde et une dialkyl- ou trialkylxanthine.

Plus particulièrement, la composition cosmétique selon l'invention permet de retarder le vieillissement de la peau qu'il soit photo-induit ou chronologique.

On sait en effet qu'une exposition prolongée au soleil provoque et accroît la formation de rides, en particulier sur le visage et a tendance à provoquer un dessèchement et une certaine rugosité de la peau.

Dans le brevet US n° 4.603.146, il a été proposé une méthode pour retarder la perte d'élasticité de la peau consistant à appliquer sur celle-ci une composition contenant une quantité efficace d'acide de vitamine A ou acide rétinoïque dans un véhicule émollient approprié.

Dans la demande de brevet EP n° 253.393, il a par ailleurs été proposé l'emploi d'autres composés du type de la vitamine A et de ses dérivés plus connus sous le terme de "rétinoïdes".

Bien qu'à l'origine ce terme ne couvrait que la vitamine A ou rétinol et ses dérivés tels que l'aldéhyde de la vitamine A ou rétinal ou encore l'acide de vitamine A ou acide rétinoïque, celui-ci englobe maintenant un grand nombre de composés qui sont recouverts par ce terme du fait qu'ils présentent des propriétés biologiques identiques ou similaires à celles de la vitamine A et de ses dérivés.

Les dérivés de xanthine tels que la caféine ou la théophylline sont généralement utilisés dans des compositions anti-cellulitiques, ceci en vue de diminuer les surcharges graisseuses et ainsi améliorer l'aspect esthétique des personnes.

Il a été notamment décrit dans la demande DE 1.962.999, une composition à administration orale destinée à traiter des désordres lipidiques liés au vieillissement comprenant l'association d'une hormone, de vitamines, de procaïne et de chloroplaste ainsi qu'éventuellement de théophylline.

Il a également été décrit dans la demande DE 1.810.705, un procédé de stabilisation de vitamines et notamment de la vitamine A, par un dérivé de la xanthine substitué en position 1, 3 ou 7 par un reste hydrocarboné acyclique en $C_3$-$C_{20}$, ainsi que des compositions pour une administration orale ou rectale.

Après d'importantes recherches et de façon suprenante, on a constaté qu'en associant un rétinoïde et un dérivé de xanthine, on obtenait une synergie particulièrement prononcée de l'effet anti-vieillissement de la peau.

Les différentes études qui ont été réalisées sur les compositions selon l'invention ont permis de mettre en évidence de façon non contestable cette synergie. Celles-ci ont été effectuées en mesurant la perte insensible en eau (PIE) dont on sait qu'elle est bien correlée avec l'activité kératolytique des rétinoïdes et qu'il a été suggéré que la mesure de la PIE pouvait être un test in vivo pour comparer l'efficacité des rétinoïdes (voir P. ELIAS, Journal of the American Academy of Dermatology, 1986, Vol. 15, n° 4 pp.797-809).

La présente invention a donc pour objet une composition cosmétique synergétique pour lutter contre le vieillissement de la peau, en particulier de la peau du visage, en vue de lui redonner et maintenir sa fermeté et son élasticité, en retardant et en inversant la perte en fibres de collagène, ladite composition contenant en association, dans un véhicule cosmétique approprié, au moins un composé du type rétinoïde et au moins une dialkyl- ou trialkylxanthine.

Comme ceci a été indiqué ci-dessus, les rétinoïdes constituent une classe de composés connus qui comprend non seulement le rétinol et l'acide rétinoïque, mais également d'autres composés chimiques qui, du fait de la similarité de leurs propriétés biologiques proches de celles de la vitamine A, sont également considérés comme étant des rétinoïdes.

Voir à ce sujet les articles suivants :

- Modulation of Cellular Interactions by Vitamin A and derivatives (Retinoïds) De Luca et al., Eds, Vol. 359 of Annals of the New-York Academy of Sciences (1981),
- "Structure-Acvitity Relationships of Retinoïds in Hamster Tracheal Organ Culture" B.L. Newton et al. , Cancer Research, 40:3413-3425 (Octobre 1980),
- "Arotinoïds, a New Class of Highly Active Retinoïds", P. Loeliger et al., European Journal of Medicinal Chemistry, 15:9-15 (1980),
- "Introduction : What is a retinoïd ?" M.B. Sporn et al., from 1985 Retinoïds, Differenciation and Disease, CIBA Foundation Symposium, Vol.113 pp.1-5,
- "Mechanism of Action of Retinoïds", M.B. Sporn et al. from American Academy of Dermatology Proceedings of a Symposium Topical Retinoïds : An update, Elias et al. Eds. New-York, 1986,
- "New Retinoïds With Potential Use in Humans", W. Bollag from Published Symposium Proceedings of Retinoïds : New Trends in Researh and Therapy., J.H. Saurat, Ed. pp.274-288 (Karger, Basel

1985), et

- "The Retinoïds", Sporn et al, Eds. Vol. 1, Academic Press (1984).

Parmi les composés rétinoïdes particulièrement préférés selon l'invention, on peut notamment citer la vitamine A ou rétinol, l'aldéhyde de vitamine A ou rétinal, l'acide de la vitamine A ou acide rétinoïque, en particulier l'acide all-trans rétinoïque et l'acide 13-cis rétinoïque, les dérivés de rétinol tels que l'acétate, le propionate ou le palmitate de rétinol et les rétinoïdes décrits dans les demandes de brevets suivantes : FR 2.570.377, EP 199.636, EP 325.540 et EP 402.072.

Selon l'invention, la concentration en rétinoïde peut varier entre 0,00001 et 10 % en poids par rapport au poids total de la composition. Toutefois, cette concentration peut varier en fonction du rétinoïde utilisé.

Ainsi, lorsque le rétinoïde est le rétinol, la concentration est de préférence comprise entre 0,05 et 10 % en poids et plus particulièrement entre 0,25 et 2 % en poids. Lorsque par contre le rétinoïde est l'acide rétinoïque, la concentration est de préférence comprise entre 0,001 et 0,3 % et plus particulièrement entre 0,005 et 0,1 %.

Comme mentionné ci-dessus, le dérivé de xanthine est une dialkyl- ou trialkylxanthine qui peut être représentée par la formule générale (I) suivante :

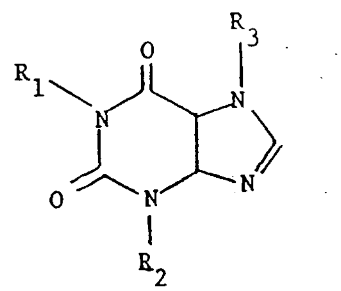

(I)

dans laquelle :

$R_1$ et $R_3$ représentent un atome d'hydrogène ou un radical alkyle en $C_1$-$C_4$, $R_1$ et $R_3$ ne pouvant toutefois représenter simultanément un atome d'hydrogène, et

$R_2$ représente un radical alkyle en $C_1$-$C_4$.

Parmi les composés de formule genérale (I), on peut notamment citer la 1,3-diméthylxanthine ou théophylline la 3,7-diméthylxanthine ou théobromine, la 1,3,7-triméthylxanthine ou caféine et la 1,7-diméthylxanthine ou paraxanthine.

Selon l'invention, la dialkyl- ou trialkylxanthine est généralement présente à une concentration comprise entre 0,1 et 5 % en poids et de préférence entre 1 et 3 % en poids par rapport au poids total de la composition.

Il convient toutefois, pour une bonne efficacité de la composition et en particulier pour un bon effet de synergie, que le rapport entre le composé de type rétinoïde et le dérivé de xanthine soit compris entre 0,00001:5 et 10:0,1, le rapport préféré étant de 0,05:3 à 10:1.

Les compositions cosmétiques selon l'invention peuvent se présenter sous diverses formes, notamment sous forme de lotions, d'émulsions, de gels anhydres ou hydroalcooliques, de gels émulsionnés, de mousses aérosols ou encore sous forme de vésicules lipidiques à base de lipides ioniques (liposomes) et/ou de lipides non ioniques.

Lorsque les compositions se présentent sous forme d'une lotion, c'est-à-dire plus particulièrement d'une solution aqueuse, elles contiennent un agent solubilisant tel que par exemple du benzoate de sodium ou encore du salicylate de triéthanolamine dans une proportion comprise entre 0,5 et 30 % en poids.

Lorsque les compositions selon l'invention se présentent sous forme d'une émulsion, elles contiennent en plus du rétinoïde et du dérivé de xanthine :

- de 3 à 50 % en poids d'au moins une huile végétale, minérale ou synthétique,
- de 1 à 10 % d'un alcool gras,
- de 0,5 à 8 % d'un acide gras en $C_8$-$C_{18}$,
- de 0,5 à 12 % d'un agent émulsionnant anionique, non ionique ou cationique, le reste étant constitué par de l'eau.

Parmi les huiles végétales, on peut citer par exemple l'huile d'amande douce, l'huile d'avocat, l'huile de coco, l'huile de germe de blé, l'huile de maïs, l'huile d'olive, l'huile de palme, l'huile de sésame, l'huile de soja, l'huile d'argan, l'huile d'onagre, l'huile de bourrache, les huiles essentielles et les cires végétales.

Parmi les huiles minérales, on peut citer par exemple l'huile de vaseline, et parmi les huiles synthétiques, les palmitates d'éthyle et d'isopropyle, les myristates d'alkyle tels que le myristaste d'isopropyle, de butyle, de cétyle le stéarate d'hexyle, les triglycérides des acides octanoïque et décanoïque (par exemple le produit vendu sous la dénomination de "MIGLYOL"® par la Société DYNAMIT NOBEL), le ricinoléate de cétyle, l'octanoate de stéaryle (huile de purcellin) et le polyisobutène hydroxylé.

Parmi les alcools gras, on peut citer l'alcool cétylique, l'alcool stéarylique, l'alcool myristique, l'alcool hydroxystéarylique, l'alcool oléique, l'alcool isostéarylique, l'alcool laurylique, l'alcool hexadécylique, l'alcool ricinoléylique, l'alcool béhénylique, l'alcool érucylique et le 2-octyl dodécanol.

Parmi les acides gras, on peut mentionner l'acide stéarique, l'acide myristique, l'acide palmitique, l'acide oléique, l'acide linoléique, l'acide laurique, l'acide isostéarique, l'acide hydroxystéarique, l'acide linolénique, l'acide ricinoléïque, l'acide arachidique, l'acide béhénique, l'acide érucique et les acides lanoliques.

Parmi les agents émulsionnants du type anionique, non-ionique ou cationique, ceux particulièrement préférés sont : les mono- et diesters du glycérol polyoxyéthylénés ou non, les esters du sorbitan polyoxyéthylénés ou non, les esters du sorbitan polyoxyéthylénés ou non, commercialisés sous les dénominations de "TWEEN"® et de "SPAN"® par la Société I.C.I., les alcools gras polyoxyéthylénés vendus sous la dénomination de "BRIJ"® par la Société I.C.I. et les acides gras polyoxyéthylénés vendus sous la dénomination de "MIRJ" par la Société I.C.I..

Lorsque les compositions se présentent sous forme d'un gel limpide, elles peuvent être soit anhydre, soit hydroalcoolique.

Lorsque les gels sont sous forme anhydre, ils contiennent de 5 à 90 % d'un alcool tel que l'éthanol et de 0,2 à 5 % d'un agent gélifiant.

Parmi les agents gélifiants, on peut notamment mentionner les dérivés de la cellulose tels que l'hydroxyéthylcellulose.

Lorsque les gels se présentent sous forme hydroalcoolique, ils contiennent :

- de 5 à 70 % d'un alcool tel que l'éthanol, et
- de 0,2 à 3 % d'un polymère carboxyvinylique tel que ceux vendus sous les dénominations de "CARBOPOL 934, 940 et/ou 941"®, préalablement neutralisés, le reste étant constitué par de l'eau.

Les compositions selon l'invention peuvent également contenir d'autres ingrédients conventionnels pour ces différents types de compositions tels que des parfums, des colorants, des agents conservateurs, des émollients, des silicones, des antioxydants, des filtres U.V., etc.

La présente invention a également pour objet un procédé de traitement de la peau en vue d'améliorer son aspect esthétique et retarder son vieillissement, en particulier de la peau du visage, ce traitement consistant à appliquer sur le visage, deux fois par jour, une quantité suffisante d'une composition cosmétique selon l'invention.

De façon générale, la durée du traitement est variable mais celui-ci donne des résultats tout à fait satisfaisants lorsqu'il est effectué pendant une période de 4 à 8 semaines, à raison de deux applications journalières.

On va maintenant donner à titre d'illustration et sans aucun caractère limitatif, plusieurs exemples des compositions cosmétiques selon l'invention.

EXEMPLES DE COMPOSITIONS COSMETIQUES

Exemple 1

| Crème de soins sous forme d'une émulsion Huile-dans-l'Eau | |
|---|---|
| Rétinol | 0,25 g |
| 1,3,7-triméthylxanthine (caféine) | 3,00 g |
| Stéarate de glycérol | 2,00 g |
| Monostéarate de sorbitan à 20 moles d'oxyde d'éthylène (TWEEN 80)® | 1,00 g |
| Alcool cétylique | 0,50 g |
| Acide stéarique | 1,40 g |
| Triéthanolamine | 0,70 g |
| Perhydrosqualène de synthèse | 24,00 g |
| Antioxydants | 0,10 g |
| Polymère carboxyvinylique neutralisé par de la triéthanolamine "CARBOPOL 940"® | 0,40 g |
| p.Hydroxybenzoate de méthyle | 0,5 g |
| Eau | qsp 100,00 g |

Exemple 2

| Crème de soins sous forme d'une émulsion Eau-dans-l'Huile | |
|---|---|
| Rétinol | 2,00 g |
| 1,3,7-triméthylxanthine (caféine) | 1,00 g |
| Monostéarate de sorbitan | 5,00 g |
| Cire microcristalline | 1,00 g |
| Huile de vaseline Esters d'acides gras en $C_8$-$C_{10}$ et d'alcools | 19,00 g |
| gras en $C_{12}$-$C_{18}$ Gel de montmorillonite modifiée organiquement et d'huile | 1,00 g |
| neutre (triglycérides d'acides caprylique et caprique) | 5,00 g |
| Antioxydant | 0,10 g |
| Propylène glycol | 3,00 g |
| p.Hydroxybenzoate de méthyle | 0,4 g |
| Eau | qsp 100,00 g |

Exemple 3

| Crème de soins sous forme d'une émulsion Eau-dans-l'Huile | |
|---|---|
| Acide rétinoïque (all trans) | 0,10 g |
| 1,3,7-triméthylxanthine (caféine) | 2,00 g |
| Lanolate de magnésium | 14,00 g |
| Alcool de lanoline | 3,60 g |
| Myristate d'isopropyle | 8,00 g |
| Huile de paraffine | 30,00 g |
| Perhydrosqualène | 10,00 g |
| Ozokérite | 4,00 g |
| Antioxydants | 0,10 g |
| p.Hydroxybenzoate de méthyle | 0,3 g |
| Eau | qsp 100,00 g |

Exemple 4

| Crème de soins pour le visage | |
|---|---|
| Acide rétinoïque (13-cis) | 0,025 g |
| 1,3,7-triméthylxanthine (caféine) | 3,00 g |
| Antioxydants | 0,10 g |
| Squalène | 20,00 g |
| Myristate d'isopropyle | 5,00 g |
| Benzoate de sodium | 3,00 g |
| Sel di-sodique de l'acide éthylène di-amine tétracétique hydraté | 0,10 g |
| CARBOPOL 940® | 0,90 g |
| Stéarate de polyéthylène glycol | 3,50 g |
| Alcool cétylique | 1,21 g |
| Acide stéarique | 2,50 g |
| Conservateurs | 0,15 g |
| Eau déminéralisée | qsp 100,00 g |

Cette crème appliquée deux fois par jour pendant 6 semaines sur le visage permet un raffermissement de la peau et une diminution des rides notamment du front et du pourtour des yeux.

Exemple 5

| Dispersion vésiculaire | |
|---|---|
| Rétinol | 0,05 g |
| 1,3,7-triméthylxanthine (caféine) | 1,00 g |
| Polyglycéryl-3 cétyl ether | 3,80 g |
| Cholestérol | 3,80 g |
| Dicétyl-phosphate | 0,40 g |
| CARBOPOL 940® | 0,42 g |
| Mélange de triglycérides d'acides octanoïque et décanoïque | 15,00 g |
| Sel disodique de l'acide éthylène diamine tétracétique hydraté | 0,10 g |
| Soude 1N q.s.p. pH = 5 | |
| Conservateur | 0,30 g |
| Parfum | 0,60 g |
| Eau | qsp 100,00 g |

Exemple 6

| Lotion | |
|---|---|
| Acide rétinoïque (all trans) | 0,01 g |
| 1,3,7-triméthylxanthine (caféine) | 1,00 g |
| Alcool éthylique | 20,00 g |
| Propylèneglycol | 5,00 g |
| Eau | qsp 100,00 g |

Exemple 7

| Gel | |
|---|---|
| Rétinol | 0,50 g |
| 1,3,7-triméthylxanthine (caféine) | 1,00 g |
| Carbomer (Carbopol 980® de Goodrich) | 0,60 g |
| Alcool éthylique | 20,00 g |
| Polyéthylèneglycol 400 | 5,00 g |
| Glycérine | 5,00 g |
| Triéthanolamine | 0,60 g |
| Eau | qsp 100,00 g |

ETUDE DE L'ACTIVITE SYNERGETIQUE

Cette étude a été réalisée en mesurant la perte insensible en eau (PIE).

La mesure de la PIE se fait à l'aide d'un évaporimètre qui est un appareil qui détermine quantitativement une évaporation d'eau, c'est-à-dire un transport d'eau par diffusion, à partir ou vers des surfaces en contact avec l'atmosphère.

Le transport de l'eau par diffusion proche d'une surface (distance inférieure à 1 cm) est déterminée par la loi de FICK :

$$\frac{1}{A} \bullet \frac{dm}{dt} = -D \frac{dp}{dx}$$

où

A = aire de la surface ($m^2$)
m = poids de l'eau transportée (g)
t = temps (en heure)
D = $0,0877\ gm^{-1}h^{-1}(1,33.10^2 Pa)^{-1}$constante relative au coefficient de diffusion
p = pression partielle de la vapeur d'eau dans l'air ($1,33.10^2$Pa)
X = distance à partir de la surface (m)

Cette formule indique que le taux d'évaporation $\frac{dm}{dt}$ est proportionnel au gradient de pression partielle $\frac{dp}{dx}$ et qu'il peut donc être déterminé par la mesure de ce gradient.

L'évaporimètre emploie une association de deux capteurs. Une petite surface de la peau est délimitée par une capsule cylindrique en téflon. Le but de cette capsule est de protéger la surface de mesure des courants d'air. A deux points A et B, disposés à des distances différentes, est placé une paire de transducteurs, l'un étant un condensateur pour la mesure de l'humidité relative, l'autre une thermistance pour la mesure de la température.

A partir des signaux recueillis par ce transducteur, l'instrument calcule d'abord la pression partielle de la vapeur d'eau aux deux points A et B puis le gradient de pression partielle et finalement le taux d'évaporation (PIE).

Selon cette méthode de mesure, on a déterminé la PIE de la crème suivante (Placébo) :

| | |
|---|---|
| Perhydrosqualène | 20,00 g |
| Myristate d'isopropyle | 5,00 g |
| Triéthanolamine | 0,50 g |
| Sel disodique de l'acide éthylène diamine tétracétique hydraté | 0,10 g |
| CARBOPOL 940® | 0,40 g |
| Glycérine | 3,00 g |
| Stéarate de polyéthylène glycol à 20 moles d'oxyde d'éthylène | 1,75 g |
| Mélange de monostéarate de glycéryle et de stéarate de polyéthylèneglycol (50/50) | 0,60 g |
| Alcool cétylique | 0,60 g |
| Alcool stéarylique | 0,60 g |
| Acide stéarique | 2,50 g |
| Antioxydants | 3,10 g |
| Conservateurs | 0,19 g |
| Eau | q.s.p. 100,00 g |

Puis l'on a mesuré la PIE de cette même crème contenant 0,5 % de rétinol (crème A), puis la PIE de cette même crème mais contenant 3 % de caféine (crème B) et enfin la PIE de cette même crème contenant une association de 0,5 % de rétinol et 3 % de caféine (crème C).

Les résultats obtenus sont rassemblés dans le tableau I suivant :

TABLEAU I

| | % Rétinol | % Caféïne | PIE |
|---|---|---|---|
| Placébo | 0 | 0 | 1,3 |
| Crème A | 0,5 | 0 | 4,1 |
| Crème B | 0 | 3 | 1,4 |
| Crème C | 0,5 | 3 | 5,4 |

<u>CONCLUSION</u> :

Effet crème A : 2,8 (4,1-1,3)

Effet crème B : 0,1 (1,4-1,3)

Effet crème C : 4,1 (5,4-1,3)

La même étude comparative a été effectuée en utilisant d'une part 0,025 % d'acide rétinoïque (all trans) (crème A') et d'autre part 3 % de caféïne (crème B') ainsi que l'association de 0,025 % d'acide rétinoïque et de 3 % de caféïne (crème C').

Les résultats obtenus sont rassemblés dans le tableau II suivant :

TABLEAU II

| | % Acide rétinoïque (all trans) | % Caféine | PIE |
|---|---|---|---|
| Placébo | 0 | 0 | 1,3 |
| Crème A' | 0,025 | 0 | 6,1 |
| Crème B' | 0 | 3 | 1,4 |
| Crème C' | 0,025 | 3 | 6,6 |

<u>CONCLUSION</u> :

Effet crème A' : 4,8 (6,1-1,3)

Effet crème B' : 0,1 (1,4-1,3)

Effet crème C' : 5,3 (6,6-1,3)

Comme on peut le constater d'après les tableaux I et II, les crèmes selon l'invention, c'est-à-dire les crèmes C et C' présentent un effet synergétique prononcé puisque la valeur trouvée de PIE est supérieure à la somme des PIE du rétinol et de la caféïne d'une part, et de l'acide rétinoïque et de la caféïne d'autre part.

## Revendications

1. Composition cosmétique destinée à être appliquée sur la peau pour lutter contre son vieillissement, caractérisée par le fait qu'elle contient en association, dans un véhicule cosmétique approprié pour une application topique, au moins un rétinoïde et au moins une dialkyl- ou trialkylxanthine.

2. Composition cosmétique selon la revendication 1, caractérisée par le fait que le rétinoïde est le rétinol, l'acide rétinoïque all trans ou 13-cis, l'acétate de rétinol, le propionate de rétinol ou le palmitate de rétinol.

3. Composition cosmétique selon l'une quelconque des revendications 1 et 2, caractérisée par le fait que la concentration en rétinoïde est comprise entre 0,00001 et 10 % en poids par rapport au poids total de la composition.

4. Composition selon l'une quelconque des revendications 1 à 3, caractérisée par le fait que le rétinoïde est le rétinol et que sa concentration est comprise entre 0,05 et 10 % et de préférence entre 0,25 et 2 % en poids.

5. Composition cosmétique selon l'une quelconque des revendications 1 à 3, caractérisée par le fait que le rétinoïde est l'acide rétinoïque all trans ou 13-cis et que sa concentration est comprise entre 0,001 et 0,3 % et de préférence entre 0,005 et 0,1 %.

6. Composition cosmétique selon la revendication 1, caractérisée par le fait que la dialkyl- ou trialkylxanthine répond à la formule générale suivante :

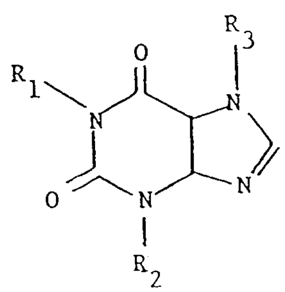

(I)

dans laquelle :

$R_1$ et $R_3$ représentent un atome d'hydrogène ou un radical alkyle en $C_1$-$C_4$, $R_1$ et $R_3$ ne pouvant toutefois représenter simultanément un atome d'hydrogène, et

$R_2$ représente un radical alkyle en $C_1$-$C_4$.

7. Composition cosmétique selon la revendication 1 ou 6, caractérisée par le fait que la dialkyl- ou trialkylxanthine est choisi dans le groupe constitué par : la 1,3-diméthylxanthine, la 3,7-diméthylxanthine, et la 1,3,7-triméthylxanthine et la 1,7 diméthylxanthine.

8. Composition cosmétique selon la revendication 1 ou les revendications 6 et 7, caractérisée par le fait que la dialkyl- ou trialkylxanthine est présente à une concentration comprise entre 0,1 et 5 % par rapport au poids total de la composition.

9. Composition cosmétique selon l'une quelconque des revendications précédentes, caractérisée par le fait que le rapport entre le rétinoïde et la dialkyl- ou trialkylxanthine est compris entre 0,00001:5 et 10:0,1.

10. Composition cosmétique selon l'une quelconque des revendications précédentes, caractérisée par le fait qu'elle se présente sous forme d'une lotion, d'une émulsion, d'un gel anhydre ou hydroalcoolique, d'un gel émulsionné, d'une mousse aérosol ou sous forme de vésicules lipidiques à base de lipides ioniques ou de lipides non ioniques.

11. Composition cosmétique selon l'une quelconque des revendications précédentes, caractérisée par le fait qu'elle contient en outre au moins un ingrédient cosmétique choisi parmi : un parfum, un colorant, un agent conservateur, un émollient, une huile de silicone, un antioxydant ou un filtre U.V.

12. Procédé cosmétique de traitement de la peau d'une personne en vue d'en améliorer son aspect esthétique et d'en retarder son vieillissement caractérisé par le fait qu'il consiste à appliquer sur la peau, notamment sur la peau du visage, une quantité suffisante d'une composition cosmétique selon l'une quelconque des revendications 1 à 11, à raison de deux applications journalières, pendant une période de 4 à 8 semaines.

## Claims

1. Cosmetic composition intended for application to the skin for combating skin ageing, characterized in that it contains, in combination, in a cosmetic vehicle suitable for topical application, at least one retinoid and at least one dialkyl- or trialkylxanthine.

2. Cosmetic composition according to Claim 1, characterized in that the retinoid is retinol, all-trans- or 13-cis-retinoic acid, retinol acetate, retinol propionate or retinol palmitate.

3. Cosmetic composition according to either of Claims 1 and 2, characterized in that the retinoid concentration is between 0.00001 and 10 % by weight relative to the total weight of the composition.

4. Composition according to any one of Claims 1 to 3, characterized in that the retinoid is retinol and in that its concentration is between 0.05 and 10 %, and preferably between 0.25 and 2 %, by weight.

5. Cosmetic composition according to any one of Claims 1 to 3, characterized in that the retinoid is all-trans- or 13-cis-retinoic acid and in that its concentration is between 0.001 and 0.3 %, and preferably 0.005 and 0.1 %.

6. Cosmetic composition according to Claim 1, characterized in that the dialkyl- or trialkylxanthine corresponds to the following general formula:

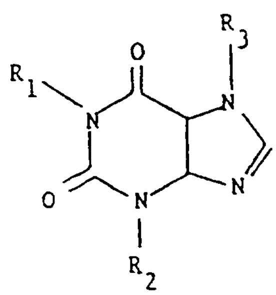

(I)

in which:

$R_1$ and $R_3$ represent a hydrogen atom or a $C_1$-$C_4$ alkyl radical, it not being possible, however, for $R_1$ and $R_3$ simultaneously to represent a hydrogen atom, and

$R_2$ represents a $C_1$-$C_4$ alkyl radical.

7. Cosmetic composition according to Claim 1 or 6, characterized in that the dialkyl- or trialkylxanthine is chosen from the group consisting of: 1,3-dimethylxanthine, 3,7-dimethylxanthine and 1,3,7-trimethylxanthine and 1,7-dimethylxanthine.

8. Cosmetic composition according to Claim 1 or Claims 6 and 7, characterized in that the dialkyl- or trialkylxanthine is present at a concentration of between 0.1 and 5 % relative to the total weight of the composition.

9. Cosmetic composition according to any one of the preceding claims, characterized in that the ratio of the retinoid to the dialkyl- or trialkylxanthine is between 0.00001:5 and 10:0.1.

10. Cosmetic composition according to any one of the preceding claims, characterized in that it takes the form of a lotion, an emulsion, an anhydrous or aqueous-alcoholic gel, an emulsified gel or an aerosol foam, or the form of lipid vesicles based on ionic lipids or on nonionic lipids.

11. Cosmetic composition according to any one of the preceding claims, characterized in that it contains, in addition, at least one cosmetic ingredient chosen from: a perfume, a colorant, a preservative, an emollient, a silicone oil, an antioxidant or a UV screening agent.

12. Cosmetic process for treating a person's skin for the purpose of improving the attractiveness of its appearance and of retarding skin ageing, characterized in that it consists in applying a sufficient amount of a cosmetic composition according to any one of Claims 1 to 11 to the skin, in particular to the skin of the face, at the rate of two applications daily for a period of 4 to 8 weeks.

**Patentansprüche**

1. Kosmetische Zubereitung für die Applikation auf der Haut zur Bekämpfung von deren Alterung, dadurch gekennzeichnet, daß sie in einem für die topische Verabreichung geeigneten kosmetischen Träger mindestens ein Retinoid zusammen mit mindestens einem Dialkyl- oder Trialkylxanthin enthält.

2. Kosmetische Zubereitung gemäß Anspruch 1, dadurch gekennzeichnet, daß das Retinoid als Retinol, als all-trans- oder 13-cis-Retinsäure, Retinolacetat, Retinolpropionat oder Retinolpalmitat vorliegt.

3. Kosmetische Zubereitung gemäß einem der Ansprüche 1 und 2, dadurch gekennzeichnet, daß die Konzentration an dem Retinoid zwischen 0,00001 Gew.-% und 10 Gew.-% in bezug auf das Gesamtgewicht der Zusammensetzung beträgt.

4. Zubereitung gemäß einem der Ansprüche 1 bis 3, dadurch gekennzeichnet, daß das Retinoid als Retinol vorliegt und seine Konzentration zwischen 0,05 Gew.-% und 10 Gew.-%, vorzugsweise zwischen 0,25 Gew.-% und 2 Gew.-%, beträgt.

5. Kosmetische Zubereitung gemäß einem der Ansprüche 1 bis 3, dadurch gekennzeichnet, daß das Retinoid als all-trans- oder 13-cis-Retinsäure vorliegt und dessen Konzentration zwischen 0,001 Gew.-% und 0,3 Gew.-%, vorzugsweise zwischen 0,05 Gew.-% und 0,1 Gew.-%, beträgt.

6. Kosmetische Zubereitung gemäß Anspruch 1, dadurch gekennzeichnet, daß das Dialkyl- oder Trialkylxanthin der folgenden allgemeinen Formel entspricht:

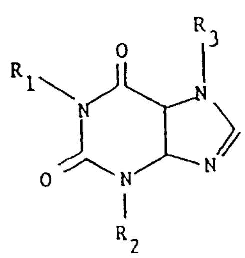

(I)

worin
$R_1$ und $R_3$ ein Wasserstoffatom oder einen $C_1$-$C_4$-Alkylrest, jedoch
$R_1$ und $R_3$ nicht gleichzeitig ein Wasserstoffatom bedeuten, sowie
$R_2$ einen $C_1$-$C_4$-Alkylrest darstellt.

7. Kosmetische Zubereitung gemäß Anspruch 1 oder 6, dadurch gekennzeichnet, daß das Dialkyl- oder Trialkylxanthin aus der aus 1,3-Dimethylxanthin, 3,7-Dimethylxanthin, 1,3,7-Trimethylxanthin oder dem

1,7-Dimethylxanthin bestehenden Gruppe ausgewählt ist.

**8.** Kosmetische Zubereitung gemäß Anspruch 1 oder den Ansprüchen 6 und 7, dadurch gekennzeichnet, daß das Dialkyl- oder Trialkylxanthin in einer Konzentration zwischen 0,1 Gew.-% und 5 Gew.-% in bezug auf das Gesamtgewicht der Zusammensetzung vorliegt.

**9.** Kosmetische Zubereitung gemäß einem der vorstehenden Ansprüche, dadurch gekennzeichnet, daß das Verhältnis zwischen dem Retinoid und dem Dialkyl- oder Trialkylxanthin zwischen 0,00001 : 5 und 10 : 0,1 beträgt.

**10.** Kosmetische Zubereitung gemäß einem der vorstehenden Ansprüche, dadurch gekennzeichnet, daß sie in Form einer Lotion, Emulsion, eines wasserfreien Gels oder eines Hydroalkoholgels, eines Emulsionsgels, eines Aerosolschaums oder in Form von Lipidvesikeln auf der Grundlage von ionischen oder nichtionischen Lipiden vorliegt.

**11.** Kosmetische Zubereitung gemäß einem der vorstehenden Ansprüche, dadurch gekennzeichnet, daß sie zusätzlich noch einen kosmetischen Hilfsstoff aus der Reihe Duftstoff, Farbstoff, Konservierungsmittel, Weichmacher, Silikonöl, Antioxidans oder einen UV-Filter enthält.

**12.** Verfahren zur kosmetischen Hautbehandlung von Personen unter dem Gesichtspunkt der Verbesserung des ästhetischen Aussehens und der Verzögerung ihrer Alterung, dadurch gekennzeichnet, daß es aus der Applikation einer ausreichenden Menge einer kosmetischen Zubereitung auf der Haut, insbesondere auf der Gesichtshaut, gemäß einem der Ansprüche 1 bis 11 besteht, und zwar in bezug auf die zweimal tägliche Anwendung während einer Zeitdauer von 4 bis 8 Wochen.